Europäisches Patentamt

European Patent Office

Office européen des brevets

(19)

(11) Veröffentlichungsnummer: **0 258 611 B1**

(12) **EUROPÄISCHE PATENTSCHRIFT**

(45) Veröffentlichungstag der Patentschrift: **11.12.91**

(51) Int. Cl.5: **C12M 1/40**, C12M 1/12

(21) Anmeldenummer: **87110723.1**

(22) Anmeldetag: **24.07.87**

(54) **Sterilisierbarer Wirbelschichtfermenter.**

(30) Priorität: **30.07.86 DE 3625698**

(43) Veröffentlichungstag der Anmeldung:
**09.03.88 Patentblatt 88/10**

(45) Bekanntmachung des Hinweises auf die
Patenterteilung:
**11.12.91 Patentblatt 91/50**

(84) Benannte Vertragsstaaten:
**AT BE CH DE FR GB IT LI NL SE**

(56) Entgegenhaltungen:
**EP-A- 0 058 426**
**US-A- 4 046 921**

**BIOTECH '83 (PROCEEDINGS OF THE INTER-
NATIONAL CONFERENCE ON THE COMMER-
CIAL APPLICATIONS OF BIOTECHNOLOGY),
4-6 May 1983, Seiten 597-610, Northwood,
GB; G. ODA: "Continuous alcohol fermentation technologies using immobilized yeast
cells"**

(73) Patentinhaber: **HOECHST AKTIENGESELL-
SCHAFT**
**Postfach 80 03 20**
**W-6230 Frankfurt am Main 80(DE)**

(72) Erfinder: **Buchholz, Rainer, Dr.**
**Grosser Ring 38**
**W-5239 Unnau-Korb(DE)**
Erfinder: **Deger, Hans-Matthias, Dr.**
**Am Rheingauer Weg 8**
**W-6238 Hofheim am Taunus(DE)**
Erfinder: **Voelskow, Hartmut, Dr.**
**Akazienstrasse 22**
**W-6234 Hattersheim am Main(DE)**
Erfinder: **Woernle, Rolf, Dr.**
**Hubertusstrasse 13**
**W-6232 Bad Soden am Taunus(DE)**

## Beschreibung

Gegenstand der Erfindung ist ein sterilisierbarer Wirbelschichtfermenter zur Durchführung von wasserarmen Fermentationen, bei denen unter sterilen Bedingungen im zunächst als Blasensäule betriebenen Wirbelschichtfermenter selbst angezüchtete und in ein wirbelfähiges Granulat überführte Mikroorganismen eingesetzt werden.

Wirbelschichtfermenter finden in biotechnologischen Verfahren vielfältige Anwendungen. So wird in der Europäischen Patentanmeldung 58 426 ein Verfahren zur Herstellung von Ethanol durch Fermentation von Kohlenhydraten beschrieben, bei dem zur Ethanolproduktion geeignete Mikroorganismen in Form rieselfähiger Partikel in ein mit Gas fluidisiertes Bett gegeben und eine oder mehrere fermentierbare Kohlenhydrate enthaltende wäßrige Nährlösungen in das Bett der fluidisierten Partikel geleitet und darin fermentiert werden. Dabei werden die Gärungsbedingungen im wesentlichen durch die Temperatur und den Sauerstoffpartialdruck des als kontinuierliche Phase in das Wirbelbett strömenden Gases eingestellt. Ein derartiges Verfahren ermöglicht die schlempefreie Herstellung von Ethanol. Durchgeführt wird dieses Verfahren in einem Fermenter, der zu Beginn mit den rieselfähigen Mikroorganismen-Partikeln beschickt und dem aus einem Vorratsbehälter eine kohlenhydrathaltige, wäßrige Nährlösung über eine Sprühdüse zugeführt wird, die über dem fluidisierten Bett angebracht ist. Das entstehende Wasser-Alkoholgemisch wird in einem Kühler kondensiert und der Fermenter mit frischem, gleichmäßig temperierten Gas versorgt.

Die vielfältigen Anwendungsmöglichkeiten von Reaktionen im Wirbelschichtfermenter zeigt die Europäische Patentanmeldung l06 l46, in der eine Vielzahl unterschiedlicher mikrobieller Metabolite und Enzyme durch fermentative Umsetzung von Präkursoren oder durch Zusatz von Effektoren auf biologischem Weg hergestellt werden. Die Mikroorganismen werden dabei in ein wirbelfähiges Granulat übergeführt. Die Präkursoren oder Effektoren werden entweder vorher in das Granulat eingearbeitet oder als Lösung oder Suspension auf das Granulat im Wirbelschichtfermenter aufgesprüht. Mit einem derartigen Verfahren lassen sich Substanzen in annehmbaren Ausbeuten herstellen, die durch chemischsynthetische Methoden nur schwer oder gar nicht zugänglich sind.

Auch in der US-Patentschrift 40 46 92l wird ein Verfahren im Wirbelschichtfermenter beschrieben, bei dem auf festen Trägern fixierte Mikroorganismen durch einen aufwärts gerichteten Gasstrom aufgewirbelt und mit einer Nährlösung besprüht werden. In dieser Patentschrift wird ausdrücklich auch auf das Problem eingegangen, daß der Mikroorganismus durch unerwünschte Fremdkeime verunreinigt sein kann. Zur Vermeidung der damit verbundenen Gefahren werden der Nährlösung fungizid oder bakterizid wirksame Substanzen zugesetzt, um das Wachstum von Mikroorganismen aus den Familien der Pseudomonadaceae, der Achromobakteriaceae, der Enterobakteriaceae, der Mikrococcen oder der Lactobacilliaceae zu verhindern. Außerdem wird empfohlen, das zur Kühlung in den Wirbelschichtfermenter eingeleitete Gas mit Schwefeldioxid zu versetzen, um dadurch unerwünschte Keime zu beseitigen. Durch diese Behandlung werden allerdings auch die für das durchzuführende Verfahren notwendigen Mikroorganismen geschädigt.

Da der Ausschluß von Fremdkeimen für die Ausbeute mikrobieller Verfahren und die Reinheit der erhaltenen Produkte entscheidend ist, stellte sich die Aufgabe, einen Wirbelschichtfermenter zu entwickeln, der sterilisierbar ist und die Durchführung von Fermentationen unter sterilen Bedingungen erlaubt.

Gegenstand der Erfindung ist deshalb ein sterilisierbarer Wirbelschichtfermenter zur Durchführung von wasserarmen Fermentationen, bei denen unter sterilen Bedingungen im zunächst als Blasensäule betriebenen Wirbelschichtfermenter selbst angezüchtete und in ein wirbelfähiges Granulat überführte Mikroorganismen eingesetzt werden, dadurch gekennzeichnet, daß

a) der Wirbelschichtfermenter (l) unten eine Öffnung (2) zum Einleiten eines Gasstromes sowie einen Anströmboden (3) mit Loch- oder Sinterplatte (4) zur Verteilung des Gasstromes aufweist,

b) seitlich am Boden des Wirbelschichtfermenters ein oder mehrere Filterelemente (5) angebracht sind, durch die die verbrauchte Kulturbrühe steril abgezogen werden kann,

c) ein Vorratsbehälter (6), versehen mit einer Dampf- (a) und einer Substratleitung (b), aus dem durch eine Leitung (c) und eine Düse (l6) das wirbelfähige Granulat mit Nährlösung besprüht werden kann, vorgesehen ist,

d) ein thermostatisierbarer Luftbefeuchter (7) vorhanden ist, der das Austrocknen der Mikroorganismen durch den Gasstrom verhindert und

e) eine Vorrichtung zum Einleiten von Heißdampf (Leitungen (a) und (d)) ein Mantelwärmeaustauscher oder ein Heizelement (8) zum Sterilisieren aller Anlagenteile des Wirbelschichtfermenters, die mit den Mikroorganismen in direktem Kontakt stehen, vorhanden ist.

Das Eindringen von Fremdkeimen in den Fermentationsansatz wird durch den erfindungsgemäßen Wirbelschichtfermenter (l) zunächst einmal dadurch verhindert, daß der Fermenter vor Beginn der Fermentation durch Einleiten von Heißdampf durch die Leitungen (a) und (d) oder durch Erhitzen des Fermenters durch einen Mantelwärmeaustauscher oder ein Heizelement (8) sterilisiert wird. In den so vorbereiteten Wirbelschichtfermenter wird nun nicht etwa die fertige Biomasse des in einem anderen Fermenter angezüchteten Mikroorganismus eingefüllt, was mit Sicherheit zu einer Kontamination mit Fremdkeimen führen würde, sondern der Wirbelschichtfermenter wird nur mit einer in einem Kleinfermenter herangezüchteten Impfkultur unter den üblicherweise angewendeten sterilen Bedingungen beschickt und in dem zunächst als Blasensäule betriebenen Wirbelschichtfermenter selbst der Mikroorganismus submers angezüchtet. Hierbei können Nährstoffe enthaltende Träger wie Reiskörner, Sojaschrot oder Haferflocken zugesetzt werden, auf denen die Mikroorganismen aufwachsen. Hierdurch wird die Bildung von Aggregaten und Kolonien von Mikroorganismen gefördert. Es gibt allerdings auch Mikroorganismen, die ohne derartige feste Träger Aggregate oder Kolonien bilden.

Sobald sich eine ausreichende Menge von Mikroorganismen gebildet hat, wird die verbrauchte Substratlösung über Filterelemente (5), die in der Nähe des Fermenterbodens installiert sind, entfernt. Als Filterelemente eignen sich Sinterfilter, Membranfilter oder Fuji-Blade, die falls erforderlich, ebenfalls mit Heißdampf sterilisiert werden können. Die Porengröße der Filterelemente ist so auszuwählen, daß Zellagglomerate und Zellkolonien zurückgehalten werden, aber Lösungsmittel und Einzelzellen passieren können. Mit dieser Verfahrensweise ist sichergestellt, daß der Wirbelschichtfermenter jetzt eine Kultur eines Mikroorganismus enthält, die nicht durch Fremdkeime verunreinigt ist.

Zur Durchführung der eigentlichen Wirbelschichtreaktion wird die bis zum Erreichen einer breiigen Konsistenz von der verbrauchten Kulturbrühe befreite Masse der Mikroorganismen durch Einleiten eines Gasstromes durch die Öffnung (2) von unten aufgewirbelt. Dazu wird Druckluft (e) oder Sauerstoff (f) verwendet, die zuvor durch die Keimfilter (9) oder (I0) gepreßt wurden, so daß durch den Gasstrom keine fremden Mikroorganismen in den Wirbelschichtreaktor hineingetragen werden können.

Das zum Aufwirbeln des biologischen Materials verwendete Gas verläßt den Wirbelschichtreaktor über die Membranfilterkerze (II) und kann dann über das Ventil (I2) im Kreis gefahren werden. Der Gasstrom wird dann durch die Leitung (g) über das Kreisgasgebläse (I3) in den thermostatisierbaren Luftbefeuchter (7) geleitet und dann das abgekühlte und mit Feuchtigkeit gesättigte Gas durch die Leitung (h) erneut über die Öffnung (2) in den Fermenter eingeleitet. Alternativ kann der Gasstrom auch im Durchlauf geführt werden, wobei er dann

über die Membranfilterkerze (II), Ventil (I4) und das Filterelement (I5) den Fermenter über die Abluftleitung (i) verläßt.

Die zum Wachstum während der Wirbelschichtfermentation benötigte Nährlösung befindet sich im Vorratsbehälter (6) und wird über die Leitung (c) und die Düse (I6) in fein verteilter Tröpfchenform in den Reaktor eingesprüht, es sei denn, die Mikroorganismen benutzen den Nährstoffe enthaltenden Träger, auf dem sie aufgewachsen sind, als ausschließliche Nährstoffquelle.

Besondere Vorsorge muß dafür getroffen werden, daß die Mikroorganismen nicht austrocknen. Hierfür dient sowohl der thermostatisierbare Luftbefeuchter (7) durch den sichergestellt wird, daß der eingeleitete Gasstrom mit Feuchtigkeit gesättigt ist. Darüber hinaus kann in den Fermenter, falls erforderlich, jederzeit keimfreies Wasser durch die Leitung (k) über das Filterelement (I7) und die Düse (I8) zugeführt und damit die Mikroorganismen befeuchtet werden.

Während der Fermentation nimmt der $CO_2$-Gehalt des Kreisgases ständig zu und der Sauerstoffgehalt entsprechend ab. Eine konstante Zusammensetzung des Gases ist aber für eine optimale Fermentation Voraussetzung. Deshalb ist der erfindungsgemäße Wirbelschichtfermenter mit (nicht gezeichneten) Kontrollelementen ausgestattet, die die kontinuierliche Entfernung des Kohlendioxids über die Membranfilterkerze (II) und das Filterelement (I5) regeln. Die ausgeschleusten Gasmengen werden durch Zuführung von Sauerstoff oder Luft über die untere Öffnung (2) ersetzt.

Bei Wirbelschichtreaktionen muß mit besonderer Sorgfalt darauf geachtet werden, daß die entstandene Reaktionswärme abgeführt wird. Deshalb wird der durch die untere Öffnung (2) eintretende Gasstrom bei seiner Passage durch den thermostatisierbaren Luftbefeuchter (7) abgekühlt. Außerdem ist der Wirbelschichtfermenter (I) von außen mit einer Mantelkühlung versehen. Falls auch dadurch noch keine ausreichende Kühlung des biologischen Materials erreicht wird, wird die überschüssige Wärme durch Verdunstungskühlung abgeführt. Die dadurch verlorengehende Wassermenge wird durch den Zusatz von keimfreiem Wasser über die Düse (I8) wieder ergänzt.

Die nachfolgenden Beispiele zeigen Anwendungsmöglichkeiten des erfindungsgemäßen Wirbelschichtfermenters:

Beispiel I

Eine Kultur des Stammes Streptomyces griseus DSM 40693 wurde in einem Kleinfermenter mit I5 I Inhalt in 3 Tagen zu einer gut gewachsenen Impfkultur herangezüchtet. Das Medium enthielt 60 g Caseinpepton, 60 g Fleischextrakt, 7,5 g Hefeextrakt und 37,5 g Natriumchlorid. Die Bebrütung erfolgte bei 35°C mit 7,5 l Luft/min und einer Rührerdrehzahl von 500 UpM bei pH 7,2. Diese Kultur wurde zur Beimpfung des erfindungsgemäßen Wirbelschichtfermenters verwendet, der zuvor 30 min bei 121°C in Anwesenheit eines Nährmediums sterilisiert worden war, das 3,4 kg Caseinpepton, 1,2 kg Fleischextrakt, 150 g Hefeextrakt und 750 g Natriumchlorid in 250 l Wasser enthielt. Eine getrennt hergestellte und sterilisierte Lösung von 3 kg Glucose, 3 kg Maltodextrin aus Maisstärke und 35 l Wasser wurde vor der Beimpfung in den Gesamtansatz gegeben.

Danach wurde der Wirbelschichtfermenter beimpft und mit 300 l/min Belüftung als Blasensäule betrieben, wobei die Temperatur auf 35°C und der pH-Wert zwischen 6,8 und 7,5 gehalten wurde.

Innerhalb von 48 Stunden errreichte die Biomasse des Streptomyces griseus ein Trockengewicht von 3,72 kg. Danach wurde über die Filterelemente (4) so viel Flüssigkeit abgesaugt, daß noch 30 l im Kulturgefäß verblieben. Diese Flüssigkeit bildete mit dem Zellhaufen des Streptomyces griseus eine breiige Masse, die durch entsprechende Erhöhung des Lufteintrags verwirbelt wurde. Über einen Zeitraum von weiteren 48 Stunden wurde pro Stunde 1 l einer sterilen Lösung bestehend aus 5 % Caseinpepton, 4 % Glucose und 4 % Maltodextrin in die wirbelnde Kulturmasse eingesprüht. Die Biomasse des Streptomyces griseus wuchs in diesen 48 Stunden auf 5,63 kg Trockengewicht an.

Beispiel 2

Eine Kultur des Stammes Streptomyces griseus DSM 40693 wurde wie im Beispiel 1 im Kleinfermenter vorgezüchtet und als Impfstoff für den erfindungsgemäßen Wirbelschichtfermenter verwendet. In diesem wurde zuvor ein Nährmedium bestehend aus 6 kg Sojamehl und 1,5 kg zerkleinerten Haferflocken in 250 l Wasser angesetzt und 45 min bei 121°C sterilisiert. Das Nährmedium wurde nach dem Abkühlen 40 Stunden auf einer Temperatur von 30°C gehalten und dann nochmals 45 min bei 121°C sterilisiert. Dann wurde eine getrennt hergestellte und sterilisierte Lösung von 1,5 kg Glucose und 3 kg Maltodextrin aus Maisstärke in 35 l Wasser zugegeben und der Ansatz beimpft. Die Kulturbedingungen waren die gleichen wie im Beispiel 1.

Im Zeitraum von 48 Stunden wuchs die Kultur zu einer guten Myzeldichte heran. Der Sedimentwert (PMV) stieg dabei von 6 % auf 15 % an. Aus der Kultur wurde wie im Beispiel 1 Flüssigkeit entzogen bis auf einen breiigen Rest von 60 l. Diese Masse wurde als Wirbelschicht weiter bebrütet. Das Trockengewicht der Biomasse (Bestimmung durch 10 minütige Zentrifugation eines Aliquots bei

4000 UpM) betrug 17,8 kg und stieg in den folgenden 48 Stunden auf 26,4 kg an (einschließlich der ungelösten Bestandteile des Nährmediums). Das Nährmedium und die Behandlung der Biomasse waren in diesem Zeitraum die gleichen wie im Beispiel 1.

Beispiel 3

Eine Kultur des Pilzstammes Penicillium roqueforti DSM 1079 wurde als Vorkultur in einem Kleinfermenter mit 15 l Inhalt herangezüchtet. Das Nährmedium enthielt 300 g Malzextrakt, 30 g Hefeextrakt, 150 g Glucose und 7,5 g $(NH_4)_2HPO_4$. Die Kulturzeit betrug 4 Tage bei 23°C mit einer Belüftung von 7,5 l/min und einer Rührerdrehzahl von 300 UpM. Der pH-Wert wurde zwischen 6,4 und 6,8 gehalten. Vor der Überimpfung dieser in den erfindungsgemäßen Wirbelschichtfermenter wurde in diesem ein Nährmedium bestehend aus 12 kg Malzextrakt, 900 g Hefeextrakt und 600 g $(NH_4)_2HPO_4$ in 230 l Wasser 30 min bei 121°C sterilisiert. 6 kg Glucose wurden getrennt in 35 l Wasser gelöst, sterilisiert und zu dem Gesamtansatz zugegeben. Nach der Beimpfung des Wirbelschichtfermenters wurde dieser 72 Stunden lang bei 23°C mit einer Belüftung von 180 l Luft/min als Blasensäule betrieben. Der pH-Wert wurde zwischen 6,4 und 6,8 gehalten. Nach Ablauf der ersten 24 Stunden wurde zu der Kultur ein Konzentrat bestehend aus 6 kg Glucose und 3 kg Malzextrakt in 20 l Wasser gleichmäßig zudosiert. Die Pilzbiomasse wuchs in den 72 Stunden auf 17,3 kg Trockenmasse an. Danach wurde die Flüssigkeit wie im Beispiel 1 abgesaugt und ein breiiger Rest von 60 l im Reaktor belassen. Dieser wurde als Wirbelschicht 96 Stunden bebrütet. In dieser Zeit wurde eine Lösung bestehend aus 6 % Malzextrakt, 6 % Glucose und 0,3 % $(NH_4)_2HPO_4$ in den Reaktor in einer Menge von 1 l/Stunde eingesprüht. Das Pilzmyzel wuchs dabei auf eine Trockenmasse von 24,5 kg weiter an.

Beispiel 4

Eine Vorkultur des Stammes Penicillium roqueforti DSM 1079 wurde wie in Beispiel 3 beschrieben vorbereitet und zur Beimpfung des vorher sterilisierten Wirbelschichtfermenters verwendet. Das im Wirbelschichtfermenter zubereitete Nährmedium enthielt in 250 l Wasser 6 kg Malzextrakt, 600 g Hefeextrakt und 150 g $(NH_4)_2HPO_4$. Getrennt wurde eine Lösung von 3 kg Glucose in 35 l $H_2O$ hergestellt und sterilisiert. Dann wurde die Kultur 36 Stunden als Blasensäule betrieben. In dieser Zeit wuchs das Pilzmyzel auf 4,6 kg Trockenmasse an. Danach wurde die Flüssigkeit bis auf einen Rest von 25 l abgesaugt. Die breiige Masse wurde als Wirbelschicht über 96 Stunden weiter bebrütet. In der ersten Hälfte dieser Zeit wurden 40 kg Quark zudosiert. Der Pilz verwuchs mit dem Quark zu grobkörnigen Teilchen, die am Ende der Bebrütungszeit vollständig durchwachsen waren und das für den verwendeten Pilz typische Aroma aufwiesen.

Beispiel 5

Eine Kultur der Hefe Schizosaccharomyces pombe DSM 70577 wurde als Vorkultur in einem Kleinfermenter mit I5 I Inhalt in dem in Beispiel 3 beschriebenen Nährmedium herangezüchtet. Die Kulturzeit betrug 30 Stunden bei 35°C, in der 7,5 I Luft/min eingeleitet und einer Rührerdrehzahl von 500 UpM angewendet wurden. Der pH-Wert wurde auf 5,6 bis 6,0 gehalten. Diese Kultur wurde dann in den vorher sterilisierten erfindungsgemäßen Wirbelschichtfermenter übergeimpft. Der Wirbelschichtfermenter enthielt ein Nährmedium bestehend aus 21 kg Malzextrakt, 3 kg Fleischextrakt und I,5 kg Caseinpepton in 285 I Wasser, das bei I21°C 30 min sterilisiert worden war. Diese Kultur wurde dann 48 Stunden bei 35°C als Blasensäule betrieben, wobei I80 I Luft/min eingeleitet und ein pH-Wert zwischen 5,6 und 6,0 aufrecht erhalten wurde. In dieser Zeit wurde eine sterilisierte heiße Lösung bestehend aus 3 kg Glucose in I0 I Wasser kontinuierlich zugegeben. Die Hefemasse wuchs in dieser Zeit auf ein Trockengewicht von 5,8 kg an. Anschließend wurde über Sterilfilter die verbrauchte Kulturbrühe bis auf einen Rest von 30 I abgesaugt. Der noch dünnflüssige Brei wurde als Wirbelschicht weiterbebrütet. Der Luftdurchsatz wurde so weit erhöht, daß eine gute Verwirbelung stattfand. Während weiterer 48 Stunden wurde I I/Stunde einer sterilen Lösung von 20 % Malzextrakt, 6 % Glucose und 3 % Caseinpepton in den Reaktor eingesprüht. Die Hefebiomasse wuchs in dieser Zeit auf ein Trockengewicht von II,8 kg weiter an.

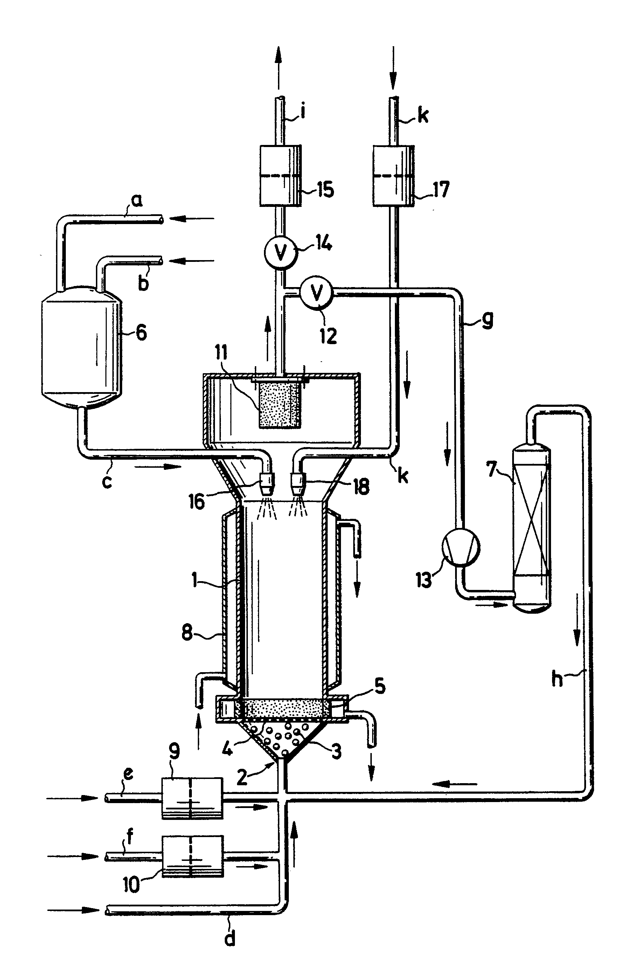

## Patentansprüche

1. Sterilisierbarer Wirbelschichtfermenter zur Durchführung von wasserarmen Fermentationen, bei denen unter sterilen Bedingungen im zunächst als Blasensäule betriebenen Wirbelschichtfermenter selbst angezüchtete und in ein wirbelfähiges Granulat überführte Mikroorganismen eingesetzt werden, dadurch gekennzeichnet, daß

    a) der Wirbelschichtfermenter (I) unten eine Öffnung (2) zum Einleiten eines Gasstromes sowie einen Anströmboden (3) mit Loch- oder Sinterplatte (4) zur Verteilung des Gasstromes aufweist,

    b) seitlich am Boden des Wirbelschichtfermenters ein oder mehrere Filterelemente (5) angebracht sind, durch die die verbrauchte Kulturbrühe steril abgezogen werden kann,

    c) ein Vorratsbehälter (6), versehen mit einer Dampf-(a) und einer Substratleitung (b), aus dem durch eine Leitung (c) und eine Düse (I6) das wirbelfähige Granulat mit Nährlösung besprüht werden kann, vorgesehen ist,

    d) ein thermostatisierbarer Luftbefeuchter (7) vorhanden ist, der das Austrocknen der Mikroorganismen durch den Gasstrom verhindert und

    e) eine Vorrichtung zum Einleiten von Heißdampf, ein Mantelwärmeaustauscher oder ein Heizelement (8) zum Sterilisieren aller Anlagenteile des Wirbelschichtfermenters, die mit den Mikroorganismen im direkten Kontakt stehen, vorhanden ist.

2. Wirbelschichtfermenter nach Anspruch I, dadurch gekennzeichnet, daß der zum Aufwirbeln der Mikroorganismen durch die untere Öffnung (2) eingeführte Gasstrom im Kreis gefahren wird.

3. Wirbelschichtfermenter nach Anspruch I, dadurch gekennzeichnet, daß der zum Aufwirbeln der Mikroorganismen durch die untere Öffnung (2) eingeführte Gasstrom im Durchlauf gefahren wird.

4. Wirbelschichtfermenter nach Anspruch I, dadurch gekennzeichnet, daß bei der Fermentation gebildete Gase durch die Membranfilterkerze (II), das Ventil (I4) und das Filterelement (I5) ausgeschleust werden.

5. Wirbelschichtfermenter nach Anspruch 3, dadurch gekennzeichnet, daß die ausgeschleusten Gasmengen durch die Zuführung von Sauerstoff oder Luft über die untere Öffnung (2) ersetzt werden.

## Claims

1. A sterilizable fluidized bed fermenter for carrying out low-moisture fermentations, in which use is made of microorganisms which are cultured under sterile conditions in the fluidized bed fermenter itself, which is initially operated as a bubble column, and are converted into fluidizable granules, which has the following features

    a) the fluidized bed fermenter (1) has at the bottom an orifice (2) for introducing a stream of gas, and an inlet bottom section (3) with a perforated or sintered disk (4) for distributing the stream of gas,

b) one or more filter elements (5) are mounted on the side of the bottom part of the fluidized bed fermenter and can be used to draw off the used culture broth under sterile conditions,

c) there is provision of a reservoir (6) which is provided with a steam line (a) and a substrate line (b), from which the fluidizable granules can be sprayed with nutrient solution through a line (c) and a nozzle (16),

d) there is a temperature-controllable air humidifier (7) which prevents the microorganisms being dried out by the stream of gas, and

e) there is a device for introducing superheated steam, a jacket heat-exchanger or a heating element (8) for sterilizing all the fittings of the fluidized bed fermenter which are in direct contact with the microorganisms.

2. A fluidized bed fermenter as claimed in claim 1, wherein the stream of gas introduced through the bottom orifice (2) to fluidize the microorganisms is recirculated.

3. A fluidized bed fermenter as claimed in claim 1, wherein the stream of gas introduced through the bottom orifice (2) to fluidize the microorganisms is passed straight through.

4. A fluidized bed fermenter as claimed in claim 1, wherein the gases formed in the fermentation are removed through the membrane filter candle (11), the valve (14) and the filter element (15).

5. A fluidized bed fermenter as claimed in claim 3, wherein the amounts of gas which are removed are replaced by introducing oxygen or air through the bottom orifice (2).

**Revendications**

1. Fermenteur à lit fluidisé stérilisable destiné à la conduite de fermentations peu exigentes en eau, dans lesquelles on utilise dans des conditions stériles des micro-organismes qui ont été élevés dans le fermenteur à lit fluidisé travaillant initialement en colonne à bulles, puis transformés en un granulat résistant au lit fluidisé, caractérisé en ce que le fermenteur à lit fluidisé (1) comprend (a) en bas une ouverture (2) pour l'introduction d'un courant de gaz, ainsi qu'un fond (3) d'arrivée du courant gazeux, muni d'une plaque perforée ou frittée (4) servant à la répartition du flux gazeux,

b) latéralement à son fond, sont agencés un ou plusieurs éléments filtrants (5) à travers lesquels le bouillon de culture usé peut être évacué dans des conditions stériles,

c) un réservoir (6) muni d'une conduite de vapeur (a) et d'une conduite de substrat (b), et à partir duquel le granulat résistant au lit fluidisé peut être arrosé de solution nutritive à l'aide d'une conduite (c) et d'une buse (16),

d) un humidificateur d'air (7) thermostaté pour éviter la dessication des micro-organismes par le courant gazeux, et

e) un dispositif pour l'introduction de vapeur chaude (conduites (a) et (d)), un échangeur de chaleur sur robe ou un élément chauffant (8) servant à stériliser tous les éléments de l'installation du fermenteur à lit fluidisé qui sont en contact direct avec les micro-organismes.

2. Fermenteur à lit fluidisé selon la revendication 1, caractérisé en ce que le courant gazeux introduit à travers l'ouverture inférieure (2) pour la fluidisation des micro-organismes circule en circuit fermé.

3. Fermenteur à lit fluidisé selon la revendication 1, caractérisé en ce que le courant gazeux introduit à travers l'ouverture inférieure (2) pour la fluidisation des micro-organismes circule en circuit ouvert.

4. Fermanteur à lit fluidisé selon la revendication 1, caractérisé en ce que les gaz formés pendant la fermentation sont évacues à travers la bougie filtrante à membrane (11), la vanne (14) et l'élément filtrant (15).

5. Fermenteur à lit fluidisé selon la revendication 3, caractérisé en ce que les quantités de gaz évacué sont remplacées par l'introduction d'oxygène ou d'air, à travers l'ouverture inférieure (2).